(19) **Europäisches Patentamt**
**European Patent Office**
**Office européen des brevets**

(11) **EP 4 495 799 A1**

(12) **EUROPEAN PATENT APPLICATION**

(43) Date of publication:
**22.01.2025 Bulletin 2025/04**

(21) Application number: **24185540.2**

(22) Date of filing: **28.06.2024**

(51) International Patent Classification (IPC):
*G06F 16/36* (2019.01)  *G06F 16/901* (2019.01)
*G16H 50/70* (2018.01)  *G16H 50/80* (2018.01)
*G06N 5/025* (2023.01)  *G06N 20/00* (2019.01)

(52) Cooperative Patent Classification (CPC):
**G06N 20/00; G06F 16/367; G06F 16/9024;**
**G06N 5/025; G16H 50/70; G16H 50/80**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **21.07.2023 US 202318356832**

(71) Applicant: **Accenture Global Solutions Limited**
**Dublin 4 (IE)**

(72) Inventors:
• **BAYRAM, Eda**
  **Dublin, 4 (IE)**
• **JANIK, Adrianna**
  **Dublin, 4 (IE)**

(74) Representative: **Black, Diego**
**Withers & Rogers LLP**
**2 London Bridge**
**London SE1 9RA (GB)**

(54) **RULE-BASED HYPOTHESIS REFINEMENT FOR LINK PREDICTION SYSTEMS**

(57)    A computer-implemented method for predicting unknown triples in an input knowledge graph, comprising: receiving the input knowledge graph; automatically obtaining an ontology of the input knowledge graph; generating a list of reference triple types for the input knowledge graph based on the ontology; automatically extracting a set of semantic rules associated with the input knowledge graph; receiving a query triple instance; extracting a query triple type corresponding to the query triple instance, and a set of semantic rules based on the input knowledge graph and the ontology; generating a set of candidate triples by expanding the query triple instance based on and as restricted by the query triple type, the input knowledge graph, and the set of semantic rules; and automatically generating a ranked list of predicted unknown triples from the set of candidate triples using a pretrained link prediction circuitry.

Figure 1

## Description

### TECHNICAL FIELD

The present disclosure relates in general to the technical field of knowledge graphs, and in particular to improvement in link prediction.

### BACKGROUND

Rapid advances in data organization and storage technologies, driven by immense customer demand, have resulted in the adoption of knowledge graphs. Knowledge graphs may represent entities with disparate characteristics and complex relationships. Entities in a knowledge graph may relate to one another as triples. Each triple may include a subject entity and an object entity connected by a particular relationship. The triples in a knowledge graph may not be complete. Plausible triples that are not previously included in a knowledge graph may be predicted via intelligent link prediction processes. It may be desirable for a link prediction system to generate triples that are missing in the knowledge graph in an efficient manner for many practical applications.

### BRIEF SUMMARY

The present disclosure relates in general to the technical field of knowledge graphs, and in particular to improvement in link prediction. The various proposed method, circuitry and systems of this disclosure are configured to operate semantic analytics on an input knowledge graph to extract its ontology and derive a set of semantic rules. The extracted ontology and semantic rules are then used for an automatic candidate generation strategy based on an input query for link/relationship prediction by filtering down from possible candidate triples to a reduced set of semantically plausible candidates. The semantically plausible candidate triples may then be evaluated by a link/relationship prediction circuitry trained based on machine learning techniques. As such, the various disclosed implementations provide a refinement of the hypothesis triple set returned by the link prediction circuitry towards semantical plausibility, thereby reducing if not eliminating hallucinations (false hypotheses) in link/relationship prediction, and at the same time improving practicality of link/relationship inference and testing.

In some example implementations, a computer-implemented method for predicting unknown triples in a knowledge graph is implemented. The method may include receiving a current instance of the knowledge graph; automatically obtaining an ontology of the knowledge graph; generating a list of reference triple types for the knowledge graph based on the ontology; automatically extracting a set of semantic rules associated with the knowledge graph; receiving a query triple instance; extracting a query triple type corresponding to the query triple instance, and a set of semantic rules based on the current instance of knowledge graph and the ontology; generating a set of candidate unknown triples by expanding the query triple instance based on and as restricted by the query triple type, the current instance of the knowledge graph, and the set of semantic rules; and automatically generating a ranked list of predicted unknown triples from the set of candidate unknown triples using a pretrained link prediction circuitry.

### BRIEF DESCRIPTION OF THE DRAWINGS

The foregoing and other objects, features, and advantages for embodiments of the present disclosure will become apparent from the following more particular description of the embodiments as illustrated in the accompanying drawings, in which reference characters refer to the same parts throughout the various views. The drawings are not necessarily to scale, with emphasis instead being placed upon illustrating principles of the present disclosure.

Figure 1 is an illustration of a fragment of an example biomedical knowledge graph comprising various subject-predicate-object triples, each shown as two graphical nodes connected by a linking edge, in accordance with certain embodiments of the present disclosure. The dashed edges represent unobserved links to be queried. Different types of entities are represented by different shapes as shown in the bottom row.

Figure 2 illustrates an example schema or ontology of an example biomedical knowledge graph containing types of entities and types of relationship that may link various types of entities.

Figure 3 illustrates an example baseline pipeline for a link prediction system operating on knowledge graphs during training and test.

Figure 4 illustrates an example rule-based hypothesis refinement module integrated into a link prediction system operating on knowledge graphs for improved efficiency during testing.

Figure 5 illustrates a schematic process diagram for the symbolic pipeline of the multi-task knowledge prediction model of Figure 4 for predicting missing links between entities, in accordance with certain embodiments of the present disclosure.

Figure 6 shows a data and logic diagram illustrating an example architecture for a computer device for implementing the example knowledge graphs and knowledge graph link prediction systems and methods above.

## DETAILED DESCRIPTION

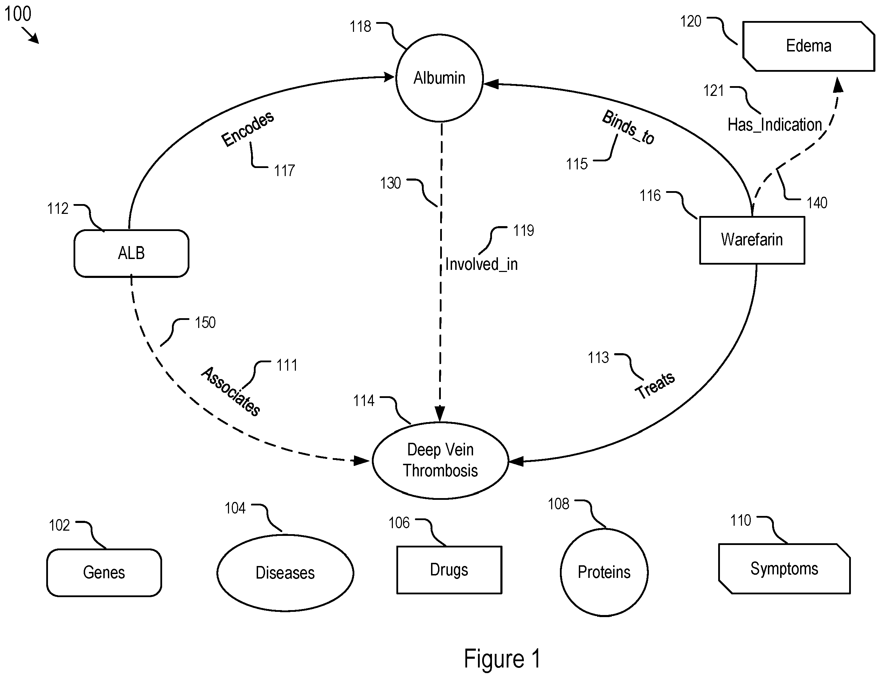

A collection of complex data items may be used for description of various sophisticated application scenarios including but not limited to community networks, social networks, general knowledge networks, industrial and enterprise operations, biomedical knowledgebase and the like. These data items may include various physical or abstract entities, concepts, and complex relationships therebetween. For example, a biomedical network may be described by various interconnecting data items in the form of entities and concepts of various types, including but not limited to genes (102), diseases (104), drugs (106), biologic molecular structures (e.g., proteins, 108), symptoms (110), medical procedures, treatment procedures, medical professionals, medical devices, medical tests, people, weather, living environment, air quality, diet, and the like. A fragment of a specific example of such biomedical network is described below in relation to Figure 1, showing various example entities/concepts and relationships therebetween.

Because of the disparity and complexity of these entity, concept, and relationship data items, a conventional relational database (e.g., in the form of data tables) may not be suitable as an efficient means for their storage and access. Instead, these data items may be defined and stored in various types of graphical databases. The collection of these data items in a graphical database may represent a graph of knowledge (alternatively referred to as a knowledge graph) that may be visualized as a web of the interconnecting entities and concepts.

Figure 1 specifically illustrates a materialized or instantiated knowledge graph 100 for the example biomedical network in accordance with certain embodiments of the present disclosure. The knowledge graph 100 may be established from known facts according to an underlying schema for the knowledge graph. Such a knowledge graph schema may function as a blueprint for expressing a set of predefined types/classes of entities, concepts, and relationships. A knowledge graph schema may be materialized or instantiated to generate an actual knowledge graph with specific entities, concepts, and relationships of the various types/classes as specified in the knowledge graph schema. A knowledge graph schema may be described in various different formats. For example, a knowledge graph schema may be constructed based on a Resource Description Framework (RDF) to provide a data model that represents the underlying knowledgebase as a collection of expressions in the form of subject-predicate-object. A subject in the schema may be any one of the predefined entity or concept types/classes whereas an object in the schema may also be any one of the predefined entity and concept types/classes. A predicate in the schema may be any one of the predefined types/classes of relationship. The term "predicate" and "relationship type" are used interchangeably in this disclosure. The term "predicate type" may be also be used to refer to "relationship type". The example knowledge graph 100 of Figure 1 may be obtained by materializing or instantiating an underlying schema with specific facts relating to entities, concepts, and relationships, where the specific entities and concepts are represented as subject or object nodes of the knowledge graph 100, and the specific relationships or predicates are represented as edges of the knowledge graph 100.

The example materialized knowledge graph 100 of Figure 1 includes various entities, concepts, and relationships of various types. For example, the knowledge graph 100 may include entities of type "gene" 102, such as "ALB" 112, entities of type "disease" 104, such as "deep vein thrombosis" 114, entities of type "drugs" 106, such as "warfarin" 116, entities of type "protein" 108, such as "albumin" 118, and entities of type "symptoms" 110, such as "edema" 120. The knowledge graph 100 may further include entity or concept relationships such as "associates" 111, "treats" 113, "binds_to" 115, "encodes" 117, is "involved_in" 119, and "has_indication" 121. While an entity as illustrated in Figure 1 may represent object of more concrete nature whereas a concept in Figure 1 represents more of an abstraction, they are not differentiated with respect to the knowledge graph data structure used in this disclosure. For simplicity, the various entities and concepts of the knowledge graph are herein collectively referred to as entities.

The knowledge graph 100 of Figure 1 essentially represents a collection of materialized subject-predicate-object triples following an underlying schema, where a subject or object may be any one of the entities (including the various concepts) represented as a set $V$, and wherein a predicate may be any one of the relationships collectively referred to as $R$, ($V$ standing for "Vertex" set of the knowledge graph, also known as nodes). For example, the knowledge graph 100 of Figure 1 includes example triples of the form "gene" (subject) "encodes" (predicate) "protein" (subject), such as "ALB" (112) "encodes" (117) "Albumin" (118). For another example, the knowledge graph 100 includes example triples of the form "protein" (subject) "binds_to" (predicate) "drug" (object), such as "albumin" (118) "binds_to" (115) "warfarin" (116). The knowledge graph 100 further includes example triples of the form "drug" (subject) "treats" (predicate) "disease" (object), such as "warfarin" (116) "treats" (113) "deep vein thrombosis" (114). The knowledge graph 100 further includes example triples of the form "gene" (subject) "associates" with (predicate) "disease" (object), such as "ALB" (112) "associates" (111) with "deep vein thrombosis" (114). The knowledge graph 100 further includes example triples of the form "protein" (subject) is "involved_in" (predicate) "disease" (object), and triples of the form "drug" (subject) "has_indication" of (predicate) "symptom" (object). The knowledge graph 100 further includes other types of entities, and other subject-predicate-object triples not illustrated in Figure 1. A knowledge graph may be referred to as KG in the disclosure below. A KG generated for biomedical purposes, such as the one of Figure 1, may be referred to as a biomedical knowledge graph.

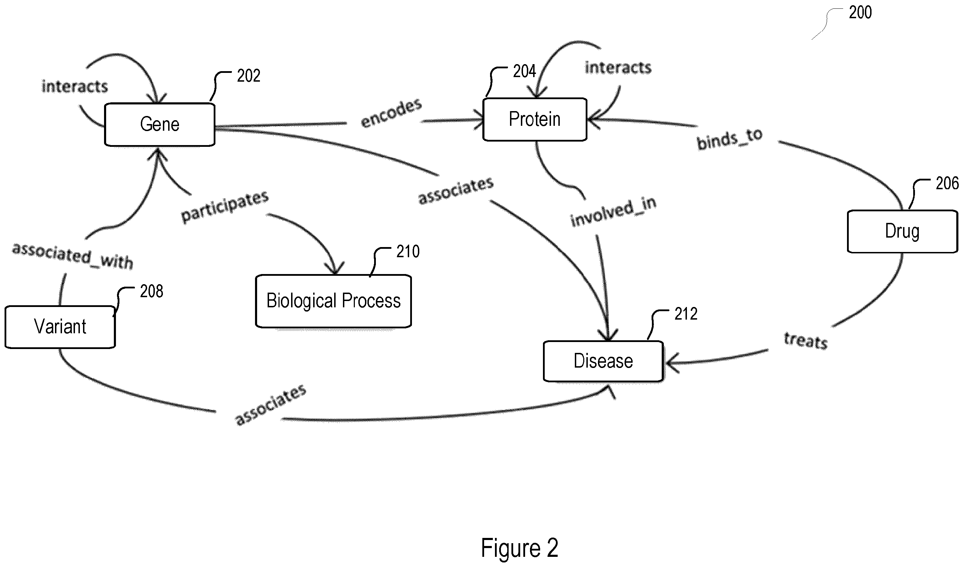

The example of Figure 1 merely represents a fragment of a biomedical knowledge graph. A practical comprehensive biologic knowledge graph may include numerous entities that are intricately related, forming a complex knowledge graph. A simplified version of a schema of an example biomedical knowledge graph is further shown in Figure 2. The biomedical knowledge graph schema 200 of Figure 2 includes several additional example entity types and their relationships. For example, the schema 200 includes these types of entities: gene 202, protein 204, drug 206, variant 208, biological process 210, and disease 212, with relationships indicated by the connecting lines/arrows. An actually instantiated knowledge graph similar to Figure 1 would include specific instances of these entity types connected to one another with the various types of connections as indicated in Figure 2.

A biomedical knowledge graph, such as the example of Figure 1 and further example that may be instantiated based on the schema of Figure 2, may be incomplete in that there may be links or relationships between the entities in the knowledge graph that may be true but missing from the facts used for knowledge graph instantiation. As an example, the triples represented by the dashed arrows 130, 140, and 150 of Figure 1 may not exist in the originally instantiated biomedical knowledge graph 100. In some example implementations, link or relationship prediction may be performed on an existing knowledge graph to uncover unknown links or relationships. The link/relationship prediction may be performed by a machine-learning model that is trained to recognize data pattern in the knowledge graph and perform intelligent generalization. For example, a link/relationship prediction system based on the machine learning model may be configured to process each of a plurality of candidate triples generated based on the knowledge graph to calculate and estimate their likelihood of being a plausible or true unknown triple of the knowledge graph. For many applications, such calculations and estimates may need to be performed in a timely manner. When a large number of candidate triples need to be processed, the link/relationship prediction system may not be able to satisfy a minimum timeliness requirement. In addition, because the large number of candidate triples may inevitably contain a significant amount of non-plausible/invalid triples, processing all of them may increase the chance for the link/relationship prediction system to inadvertently endorsing triples that cannot be true.

The various proposed method, circuitry and systems of this disclosure are configured to operate semantic analytics on an input knowledge graph to extract its ontology and derive a set of semantic rules. The extracted ontology and rules are then used for an automatic candidate generation strategy based on an input query for link/relationship prediction by filtering down from possible candidate triples to a reduced set of semantically plausible candidates. The semantically plausible candidate triples may then be evaluated by a link/relationship prediction circuitry trained based on machine learning techniques. As such, the various disclosed implementations herein provide a refinement of the hypothesis triple set returned by the link prediction circuitry towards semantical plausibility, thereby reducing if not eliminating hallucinations (false hypotheses) in link/relationship prediction and at the same time improving practicality of link/relationship inference and testing.

As described above, in a knowledge graph, data entities may be represented by a set of nodes connected by a set of relations, which may be encoded by multiple types of directed or undirected edges between the nodes. A knowledge graph may be embodied or stored as a set of triples (i.e., relational facts) $G = \{(s,p,o)\} \subseteq V \times R \times V$. Alternatively, the KG may be embodied in some other forms but these triples may nevertheless be extractable from such alternative forms of knowledge graphs. These triples may each be represented by:

$$t = (s, p, o)$$

where s represents the subject $s \in V$ and, o represents the object node $o \in V$, and *p* represents the predicate $p \in R$ which represents a directed relationship from the subject node to the object node. The term relation or relationship in this context is often referred to as predicate.

An example of a triple in a biomedical knowledge graph based on the example schema of Figure 2 may be *(MYH7, encodes, beta (β)-myosin heavy chain)* where the subject node is *MYH7*, which is a gene, and the object node is *beta (β)-myosin heavy chain*, which is a protein. This triple expresses the relational fact for a gene encoding a protein. The notation "()" in (*s,p,o*) is used to represent an actual instance of triple with s, p, and o being actual instantiated entities, rather than an entity types or predicate (the example schema of Figure 2 show types of entities and relationships).

A KG, either as initially instantiated or later supplemented, are often incomplete. In other words, they may have missing but valid/true triples. In some implementations, a link/relationship predictor (hereby referred to as link predictor for simplicity) may be developed for predicting one or more missing triples in the KG. In some further example implementations, a system may be configured to process an incomplete input triple for the link predictor to specifically generate or infer a set of hypothesis triples based on the input incomplete triple. Such an incomplete input triple, with a missing target node (either the subject node or the object node) or a missing target relation, may be referred to as a query triple, and the missing part may be represented by a question mark "?". For example, a query triple *(MYH7, encodes, ?)* inquires the object nodes directed by the node *MYH7* through 'encodes' type of relationship in the example biomedical knowledge graph above.

Different forms of query triples may correspond to link or relationship prediction. For example, a query triple for link prediction may include an entity and a relation, but leave out another entity for the prediction task. Such a query triple for link prediction may be expressed as $(?, p, o)$ for predicting the subject node, or $(s,p,?)$ for predicting the object node of a missing triple in the KG. For another example, a query triple for relation prediction may include two entities, but leave out the relation for the prediction task. Such a query triple for relation prediction may be expressed as predicting the relation type of the triple associated with a missing link between a subject and an object node in the KG, i.e., $(s,?,o)$.

In some example implementations, KG completion by a link/relation predictor may be addressed through graph machine learning approaches and notably by KG embedding learning techniques. KG embedding learning methods such as TransE, ComplEx, and RotatE may be designed to learn a vector representation, referred to as embedding, for each node and relation by being trained with the known triples observed in the input KG. In the disclosure below, the term link predictor is used to generally refer to predictors that predicts either or both of links (entities) and relations.

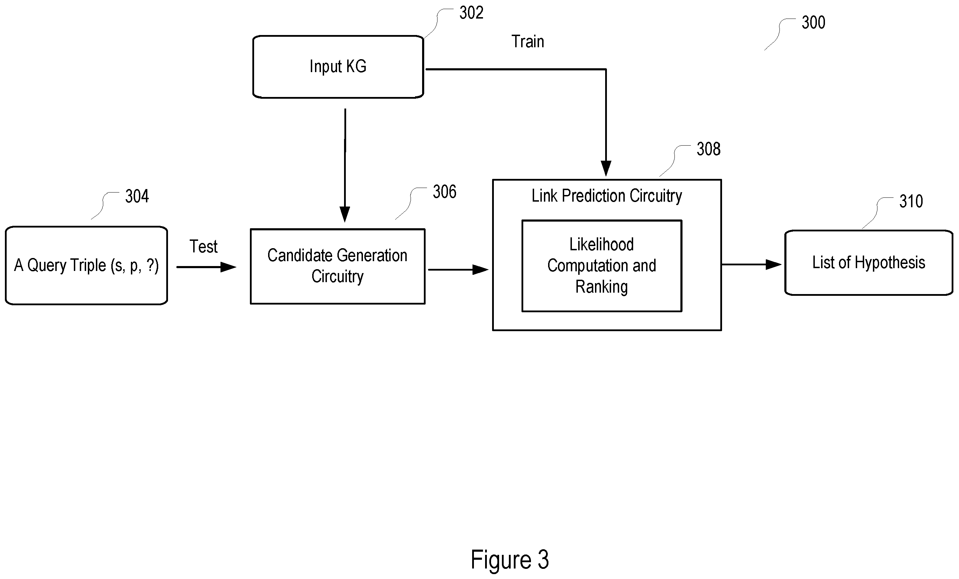

Figure 3 shows an example system 300 for link prediction by query. The system 300 may include a link predictor or link prediction circuitry 308 for receiving an input KG 302, for receiving candidate triples from a candidate generator or candidate generation circuitry 306, and for generating a list of hypothesis triples that have been unknown to the KG. The candidate generation circuitry 306 may be configured to generate the candidate triples for processing by the link prediction circuitry 308 based on a query triple 304 and the input KG 302.

In further detail, when a query triple is inquired to the link prediction system 300, a set of candidate triples may be generated by enumerating over the possible set of target nodes or target relations existing in the input KG 302. The term candidate triple is used to refer to a triple whose factuality is to be estimated by the link prediction circuitry 308. In a link prediction system, if there is no particular strategy for candidate generation, the set of candidates would consist of all possible triples with the given counterparts of the query triple. For instance, in a link prediction task represented by a query triple $(s,p,?)$, a candidate triple may be generated by replacing the target node (the question mark) with each node in KG's node set. The generated candidates, i.e., $\{(s,p,o) \,\forall o \in V\}$, may then be provided to the link prediction circuitry 308. The link prediction circuitry 308 may use a learned scoring function previously trained on the input KG 302 in order to compute the likelihood of each candidate triple. According to the computed likelihoods, the link prediction circuitry 308 returns a ranked list of candidates, which is referred to as the list of hypotheses.

The link prediction system 300 above in Figure 3 relies on the KG embedding learning frameworks trained on the input KG to learn the scoring function and to infer missing triples but without any information on the semantics or the ontology of the KG. This is because the candidate generation circuitry 306 lacks of certain candidate generation strategy.

The ontology, alternatively referred to as the schema of the KG, as described above in relation to Figure 2, consists of entity types and relation types between entity types. As such, the ontology shows which kind of triple types can be composed by a subject entity type, a relation type and an object entity type. As described in further detail below, the term "triple type" refers to types of entities and type of relationship of a triple, rather than the actual triple instance. For example, <gene, interact, gene> represents a triple type whereas (MYH7, interact, HADH) represents an actual triple instance (where MYH7 and HADH are both specific genes). Triple types and triple instances are represented by "<>" and "()" notations, respectively.

The semantics of the KG, additionally, may include various properties attached to each triple type, relaying some ontological rules. As an example, some triple types in the ontology shown in Figure 2 and their example semantics properties (e.g., directionality and cardinality) are given in the table below and explained in further detail following the table).

| Triple Type in the Ontology | Semantic Property: Directionality | Semantic Property: Cardinality | Instance |
| --- | --- | --- | --- |
| <gene, interacts, gene> | symmetric | many-to-many | (STAT1, interacts, HADH) |
| <gene, encodes, protein> | asymmetric | one-to-many | (MYH7, encodes, beta (β)-myosin heavy chain |
| <variant, associates, gene> | asymmetric | many-to-one | (rs727504237, associated_with, MYH7 |

A link prediction system that is agnostic to the ontology and the semantic rules above may be adaptable to different KGs but may also lead to several technical issues. For example, link prediction systems that lack candidate generation strategies naively compute the likelihood of any possible candidate regardless of whether they are semantically plausible or not. A semantically plausible candidate may be defined as a triple that is coherent with the ontology of the KG and also conforms to the semantic rules attached to the candidate triple type given the query triple and the input KG. Semantically invalid candidates provided to the link predictors may cause hallucinations.

For example, with respect to a biomedical KG based on the schema 200 of Figure 2, given a link prediction task associated with a query triple *(?, encodes, beta (β)-myosin heavy chain),* the semantically plausible candidate may be expected to have a subject node-target node of this link prediction task- of gene type since the object of the query is a protein type of node. This can be seen by checking the ontology or schema of the KG depicted in Figure 2, where a protein can be encoded only by the gene type of nodes. For example, according to the ontology or schema 200, the subject node cannot be a drug. As such, (warfarin, encodes, *beta (β)-myosin heavy chain*) would be a semantically implausible/invalid candidate triple as warfarin is a drug type of node.

If a semantically invalid candidate is provided to the link predictor at test time, it is ideally expected to be scored lower than plausible candidates by the trained link prediction model therein. However, as every provided candidate-semantically plausible or not-appears in the hypothesis set of the link predictor within a ranked list by their score, there is no way to tell if a hypothesis emerged from a semantically plausible candidate or not by checking its rank or score by the link predictor. This is due to the link prediction model's black-box (not interpretable) design of the learning mechanism. As such, the end user does not have access to an explanation for an output hypothesis. In addition, depending on the training process, it is not guaranteed that semantically implausible candidate would always score low by the link predictor.

As such, the lack of candidate generation strategy for the link predictor may lead to some implausible hypothesis to come forward at the output of the link predictor. In some cases, such hypothesis may even be ranked higher than the semantically plausible ones, due to, for example, a lack of semantics in learning of the scoring function during training of the model. Such false hypotheses of the link predictor may be referred to as hallucinations.

In addition, when candidate triples for a link prediction system is generated without any particular strategy and include all possible candidate triples for a query, then it may become impractical for the link predictor to effectively evaluate all of the candidate triples and further impractical for utilization of the output from the link prediction. For example, if candidate triple generation for a query triple $(s,p,?)$ is non-restrictive and includes each node in the KG, e.g., $\{(s,p,o) \; \forall o \in V\}$, then the size of the naively generated candidate set would be $|V|$ and the candidate set would include many semantically invalid triples. At inference time, the link predictor would need to evaluate and rank all of the candidates. It may be redundant and impractical to evaluate all of the semantically invalid triples. Moreover, after the evaluation of the candidates, the link predictor returns the hypothesis list to end-user. In biomedical context, the returned hypothesis may contain at least one lab-testable hypothesis. The end-user could be a clinician who is expected to test the hypothesis in a wet lab starting from the most likely triple in the list of hypotheses. A bulky list of hypotheses is troublesome due to its cost in time and labor in lab tests. The wet lab testing may include but not limited to, for example, physically extracting an entity such as particular gene in the hypothesis list to determine its expressive relationship with a particular protein.

The various additional example implementations below in the form of circuitry, systems, and methods are designed and configured to operate semantic analytics on an input knowledge graph to extract its ontology and derive a set of semantic rules. The extracted ontology and rules are then used for an automatic candidate generation strategy based on an input query for link prediction by filtering down from possible candidate triples to a reduced set of semantically plausible candidates. The semantically plausible candidate triples are then evaluated by a link prediction circuitry trained based on machine learning techniques. As such, the various disclosed implementations provide a refinement of the hypothesis triple set returned by the link prediction circuitry towards semantical plausibility, thereby reducing, if not eliminating, hallucinations (false hypotheses) in link prediction and at the same time improving practicality of link inference and testing.

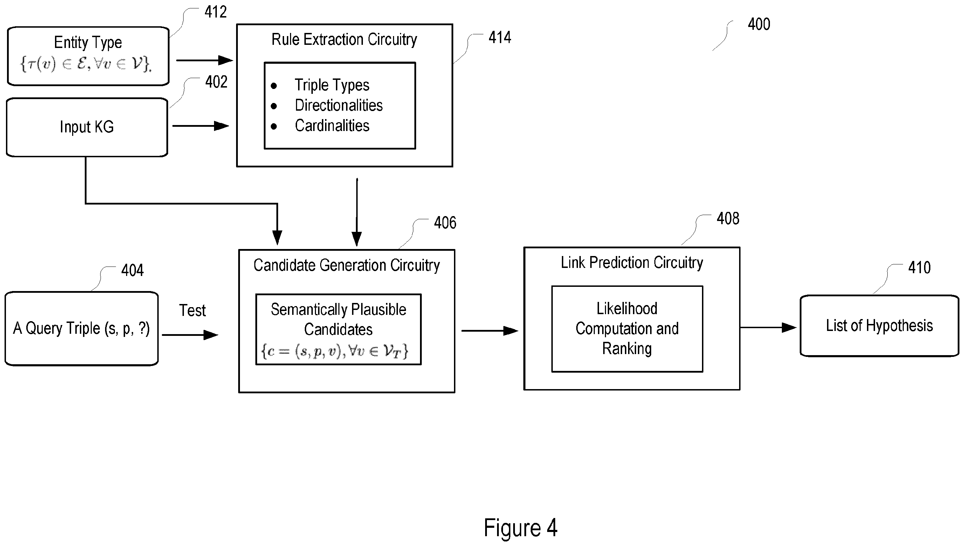

In other words, in the various example implementations below, semantic rules are extracted and integrated to a link predictor in a post-hoc manner. In other words, the semantic rules as extracted facilitate elimination of semantically invalid candidate triples and reduction of a number of candidates to semantically plausible ones that will be input into the pre-trained link predictor for prediction of missing links in the link prediction inquiry, thereby greatly reducing the number of candidates that need to be evaluated by the link predictor and the evaluation time for improving timeliness and link prediction. As shown in another example link prediction system 400 of Figure 4, a rule extraction circuitry 414 may be added to the example link prediction system shown in Figure 3. The rule extraction circuitry takes the knowledge graph 402 and/or entity type mappings 412 (if available) as input. The semantic and/or ontology rules so generated may then be fed to the candidate generation circuitry 406, which also takes as input the query triple 404 and the knowledge graph 402. The candidate generation circuitry 406 of Figure 4, unlike the candidate generation circuitry 306 of Figure 3 that generates a list of candidate triples by filling in the missing part in the input query with all possible nodes of the knowledge graph, would instead generate only semantically plausible candidates according to the query triple 404 and the semantic and ontology rules extracted from the rule extraction circuitry 414. As further shown in Figure 4, only the semantically plausible candidates as generated by the candidate generation circuitry 406 are passed to the link predictor circuitry 408, which performs likelihood computation and candidate ranking in order to return a list of hypothesis triples 410 that are missing in the knowledge graph 402.

The example implementation of Figure 4 thus performs a rule extraction (by the rule extraction circuitry 414) and candidate generation by the candidate generation circuitry 406 prior to performing link prediction by the link prediction circuitry 408. Specifically, a set of semantic/ontology rules are first extracted from the input KG containing the triples, $G = \{t = (s,p,o)| s, o \in V, p \in R\}$, and the entity type mappings of the KG nodes $\{\tau(v) \in E, \; \forall v \in V\}$. Here, the set of entity types in the KG is denoted by $E$ and a function returning the entity type of a node denoted as $\tau(v): V \rightarrow) E$ may be employed, where $v \in V$.

For instance, the entity type mapping function may return the type of the node *COVID-19* as *disease.* Then the extracted set of semantic rules and the input KG are provided using the generation of candidate triples. The candidate generation process may involve receiving a link prediction query such as $(s, p, ?)$ and generates a set of candidates, $\{(s, p, v), \forall v \in V_T\}$, for that query triple, where $V_T \subset V$ stands for the plausible set of target nodes for the query triple, as determined by the candidate generation step. Alternatively, the candidate generation process may receive a relation prediction query triple such as $(s, ?, o)$ and generate a set of candidates $\{(s, p, o), \forall p \in R_T\}$, where $R_T \subset R$ stands for the plausible set of target relations for the query triple. The rule extraction and candidate generation processes involved in Figure 4 are described in further detail below.

In some example implementations, the rule extraction circuitry or layer 414 may be configured to exploit the available entity type mappings 412 and the input KG 402 to extract semantic rules to be used by the candidate generation circuitry 406. For example, the triple types defining the ontology of the input KG 402 may be first derived. Second, certain semantic properties such as directionality and cardinality of each triple type may be further derived.

In some example implementations, the extraction of triple types or ontology may be based on entity and relation types of the triples. In other words, the type of a triple $t = (s, p, o)$ may be defined by its subject entity type, relation type and object entity type, i.e., $\langle \tau(s), p, \tau(o) \rangle$. For example, an instance of a triple type <drug, treats, disease> may be a triple t = (Erythropoietin (EPO), treats, Deep vein thrombosis (DVT)). Inversely, the triple type of the triple instance (Erythropoietin (EPO), treats, Deep vein thrombosis (DVT)) may be extracted as <drug, treats, disease> .

In some example implementations, given the input KG triples, $G$, it is possible to extract the ontology from the list of observed triple types, which may be referred to as semantically plausible triple types in this disclosure. This can be done by labelling the subject and object node of each triple instances in the KG 402 by their entity types and then counting the number of instances in each observed triple type $\langle \tau(s), p, \tau(o) \rangle | t = (s, p, o) \in E$.

Because the instances contained in the KG 402 usually do not contain all tri-combinations of entities and predicates, the number of observed triple types may be inherently much smaller than the number of all possible (entity type, predicate, entity type) combinations $|E| \times |R| \times |E|$. In other words, some possible type combinations do not exist in the ontology from which the KG 402 originates, possibly because that non-existent triple types are not semantically plausible. As such, the ontology may be derived from KG instances. Alternatively, in many example biomedical KGs, such as @PrimeKG, @Hetionet, the entity types and the ontology may be readily available in various forms and may have been used as underlying schema for instantiating the KGs. For instance, some triple types found in @Hetionet and their number of instances are given below:

| Triple types | # triples |
| --- | --- |
| <Compound,downregulates,Gene> | 21102 |
| <Disease, associates, Gene> | 12623 |
| <Gene, interacts, Gene> | 147164 |
| <Gene, participates, Pathway> | 84372 |

In some example implementations, triple types extracted above by counting but with number of triple instances that a smaller than a predefined threshold level may be removed from the list of plausible triple types. In other words, the triple types with very few corresponding triple instances in the KG may be considered as outliers and thus not part of the semantically plausible triple types.

In some other example implementations, the list of plausible triple types may be directed extracted from an underlying schema or ontology if available. Example schema or ontology such as the one shown in Figure 2 for a biomedical knowledge graph may directly contain information related to allowed or plausible triple types. In some other example implementations, the list of plausible triple types may be extracted based on both an available schema/ontology and mapping of the actual input knowledge graph. For example, the list of plausible triple types may be a combination or intersection of the lists of plausible triples derived along both extraction paths.

In some further example implementations, once semantically plausible triple types from input KG are extracted, it may be possible to further derive some semantic properties attached to each plausible triple type. A subset of semantic properties of each of the plausible triple type may be derived. For example, a number of categories of triple type properties may be focused on. Specifically, two categories of triple type properties may be derived, including directional properties and cardinal properties, as described in further detail below.

From a directionality point of view, a triple either manifests a symmetric relationship where the position of subject and object node does not matter (direction of the edge is irrelevant), or an asymmetric relationship between subject and object where the position of the subject and object matters by being strictly directional.

In a biomedical KG such as a KG based on the schema of Figure 2, triple types with symmetric relationships can be encountered, for example, in protein-protein interactions (PPI), drug-drug interactions, or gene interactions, where the edge represents symmetric mutual interactions. An example symmetric triple type may be <gene, interact, gene> with an example triple instance of (*HADH, interacts, STAT1*). The reciprocals of such triple instances are also semantically valid, e.g., triple instance (*STAT1, interacts, HADH*) is also of valid <gene, interact, gene> type.

Inherently, symmetric relationships may mostly arise in triples where the subject and object nodes are of the same entity type. But symmetric triple types are not so limited. In some situations, triples involve different types of subjects and objects may be symmetric. Furthermore, not all the triple types whose subject and the object are of the same entity types are symmetric. For instance, <gene, regulate, gene> poses a strictly directional relationship between subject and object nodes, e.g., (*HADH, regulates, STAT1*) may be a valid triple instance, but its reciprocal instance may not be valid, as STAT1 does not regulate HADH. This is because the impact between entities of the same type with respect to certain type of edges may not be symmetric.

Examples of asymmetric triple can be given from triple type <gene, regulate, gene> as mentioned above or <drug, treats, disease> with an example instance of (*warfarin, treats, deep vein thrombosis (DVT)*). Being asymmetric, the reciprocals of such triples are semantically invalid.

Designation of symmetric or asymmetric property of triples may be indicated in the KG in various manners. The rule extraction circuitry 414 thus may be configured to extract such information accordingly. In some example implementations, designation of the symmetric and asymmetric triple types from the input KG may depend on how triples are stored in the KG. In some knowledge bases, for example, triples may be stored in RDF format where all triples are directed (or directional) by default. As such, in those knowledge bases, directional properties are part of the stored triples. As such, a triple and its symmetric counterpart may be stored as separate entries in the knowledge base. In these types of KGs, if reciprocal of a triple type does not exist, then that triple type would be inferred as being asymmetric. Otherwise, the triple type would be symmetric. For instance, if the triple (*HADH, interacts, STAT1*) is in the KG together with its reciprocal (*STAT1, interacts, HADH*) then one can define the triple type <*gene, interacts, gene*> as a symmetric relation. However, it is noted that not all the reciprocal instances of the symmetric triples can be observed in the KG due to incompleteness.

In some other KGs, for symmetric triples such as (*HADH, interacts, STAT1*), the valid reciprocal instance of (*STAT1, interacts, HADH*) may be omitted in the KGs. For those KGs, if there is no prior information about the semantics, it may not be possible to define a triple type as symmetric or asymmetric just by looking at the triple instances. For those KGs, an additional semantic tagging to relation types may be required to distinguish symmetric triple types from the asymmetric ones. The rule extraction circuitry 414, thus may be configured accordingly to extract symmetry or asymmetry properties of triples from such additional semantic tagging.

In some example implementations, subject and object cardinalities as another semantic property may be attributed to a triple type. For example, given a subject node and a relation in a triple type ($s$, $p$, ?), the triple's object cardinality may be referred to as singular if the triple is valid for a unique object node, otherwise it is plural if the triple is valid for multiple distinct object nodes. Similarly, given the object and the relation of a triple type (?, $p$, $o$), the triple's subject cardinality is plural if it links to multiple distinct subject nodes, otherwise the triple's subject cardinality is singular when it links to a unique subject node. As such, in some example implementations, a triple type can be categorized with respect to their subject and object cardinalities as follows:

- *One-to-one relations*: These types of triples' subject and object cardinalities are singular. An example one-to-one triple type encountered in biomedical KGs may be <Pathway's origin, located-in, Cellular_structure>, as location of pathway's origin is usually unique with respect to cellular structures in both ways. As such, a particular pathway origin instance is located only in a unique instance of cellular structure.
- *One-to-many relations*: The subject cardinality of this type of triple is singular while the object cardinality is plural. An example one-to-many triple type in biomedical KGs may be <gene, associates, variant> with an example instance (MYH7, associates, rs727504237), since a gene can have multiple variants, yet a variant is uniquely originated from one specific gene. Another example triple type <gene, encodes, protein> also constitutes one-to-many relation with an example instance being (*MYH7, encodes, beta ($\beta$)-myosin heavy chain*), where a gene may encode multiple proteins whereas a particular protein is only encoded by one specific gene.
- *Many-to-one relations:* The object cardinality of this type of triple is singular while the subject cardinality is plural. An example many-to-one triple type encountered in biomedical KGs may be <variant, associated_with, gene> with an example instance (rs727504237, associated_with, MYH7), as multiple variants may be associated with one gene whereas a variant can only be uniquely associated with a gene instance.
- *Many-to-many relations*: The subject and object cardinality of these triple types are both plural. An example many-to-many triple type encountered in biomedical KGs may be <gene, participate, biological_process>, as a gene instance can participate in multiple biological processes, whereas a process can be participated by multiple gene instances.

In some example implementations, designation of cardinality of the triples may be obtained by checking the number of instances, subject nodes and object node of each type of triple in the KG. For instance, the number of instances of a one-to-one triple type may be equal to the number of subject nodes and number of object nodes. In general, the number of instances of a triple type whose subject cardinality is singular is equal to the number of object nodes and then the number of instances of a triple type whose object cardinality is singular is equal to the number of subject nodes. In many-to-many triple types, however, the number of instances is usually larger than the number of object or subject nodes. Some examples are given from biomedical KGs in the table below:

| Triple types | # triples | Subjects | Objects | Cardinality |
|---|---|---|---|---|
| (Pathway's origin, located_in, Cellular_Structure) | 1822 | 1822 | 1822 | one-to-one |
| (Gene, encodes, Protein) | 11571 | 1689 | 11571 | one-to-many |
| (Variant, associated_with, Gene) | 125 00 | 125 000 | 17 381 | many-to-one |
| (Gene, participates, Biological_Process) | 559504 | 14772 | 11381 | many-to-many |

In some example implementations, symmetric triple types can only be one-to-one or many-to-many. However, not every one-to-one or many-to-many triple types are symmetric. On the other hand, one-to-many and many-to-one relationships are inherently asymmetric. As such, it may be advantageous to separately derive and track the directionality and cardinality by the rule extraction circuitry 414.

In some example implementations, the output from the rule extraction circuitry 414 of Figure 4 as described above, including extracted triple types (ontology), directionality of each of the triple types, and cardinality of each of the triple types, may be input into the candidate generation circuitry 406 of Figure 4 for generation of semantically plausible candidate triples corresponding to the query triple 404. The candidate generation circuitry 406 may be configured to generate semantically plausible candidates by (1) determining the set of triple types that a link prediction query or a relation prediction query applies to; and (2) determining the target set of nodes in a link prediction query or the target set of relations in a relation prediction query, which are semantically plausible with respect to derived semantic rules. Example detailed implementations are further provided below.

In some example implementations, candidate generation circuitry 406 may be first configured to determine the set of plausible triple types that the query triple 404 (either a link prediction query or a relation prediction query) applies to and then generate a set of candidate triples by replacing the missing part (subject, object, or predicate) with a target set of nodes of the KG having a matching type for the missing part within the plausible triple types. In such a manner, the set of candidate triples may be reduced to semantically plausible ones.

Specifically, in some example implementations, the query triple 404 may have already given a hint on the triple types associated with the output link prediction hypothesis 410. For example, in case where the task is to predict the subject node for a query triple $(?, p, o)$, the relation type and the object entity type associated with the query triple, $\langle ?, p, \tau(o) \rangle$ may be checked/extracted and compared to the set of triple types (ontology) extracted from the KG 402 and/or the entity type mappings 412 by the rule extraction circuitry 414 in order to identify which semantically plausible triple types match the query triple 404 in terms of the relation type and the object entity type $\langle ?, p, \tau(o) \rangle$. The set of semantically plausible subject entity types included in the semantically plausible triple types corresponding to the query triple 404 so identified may be denoted as $E^s_{p,\tau(o)}$. Entity nodes in the KG that belonging to $E^s_{p,\tau(o)}$ entity types may then be used to replace the subject "?" in the query triple to generate the semantically plausible candidate triples, whereas other entity nodes may be considered implausible and may not be used to expand the input query triple 404 into triple candidates. The semantically plausible target set of nodes with entity types of $E^s_{p,\tau(o)}$ may be denoted as $V_T = \{ v \in V \,|\, \tau(v) \in E^s_{p,\tau(v)} \}$. The set of semantically plausible target nodes is usually a small subset of all KG nodes, i.e., $V_T \subset V$. Instead delivering all KG nodes $V$ as candidate of missing subject node in the query triple to the link predictor 408, it is possible to reduce the target set of nodes to $V_T$ using KG triple types derived in the rule extraction circuitry 414 such that only a plausible set of target node may need to be computed and evaluated by the link predictor 408. Inherently, this drops down the size of candidate node set $\{(v,p,o), \forall v \in V_T\}$ much below the size of naive set of candidates having target subject node as all KG nodes, i.e., $|V_T| \ll |V|$.

Similarly, in case where the task is to predict the object node for a query triple $(s,p,?)$, the relation type and the subject entity type associated with the query triple may be checked/extracted and compared to the set of triple types extracted from the KG 402 and the entity type mappings 412 by the rule extraction circuitry 414 in order to identify which semantically plausible triple types match the query triple 404 in terms of the relation type and the object entity type. The set of semantically plausible object entity types included in the semantically plausible triple types corresponding to the query triple 404 so identified may be denoted as $E^{o}_{\tau(s),p}$. Entity nodes in the KG that belonging to $E^{o}_{\tau(s),p}$ entity types may then be used to replace the object "?" in the query triple to generate the semantically plausible candidate triples, whereas other entity nodes may be considered implausible and may not be used to expand the input query triple 404 into triple candidates. The semantically plausible target set of nodes with entity types of $E^{o}_{\tau(s),p}$ as objects may be denoted as

$$V_T = \{v \in V | \tau(v) \in E^{o}_{\tau(s),p}\}$$

. Instead of computing the likelihood of all KG nodes $V$ as candidate of missing object link in the query tribble by the link predictor 408, it is possible to reduce the target set of nodes to $V_T$ using KG triple types derived in the rule extraction circuitry 414 such that only a plausible set of target node may need to be computed and evaluated. Inherently, this drops down the size of candidate node set $\{v,p,o)\forall v \in V_T\}$ much below the size of naive set of candidates having target object node as all KG nodes, i.e., $|V_T| « |V|$.

Similar strategy for generation of reduced number of candidates may be adopted in the relation prediction task as well. For example, semantically plausible relation types may be denoted as $R_{\tau(s),\tau(o)}$ for a triple query to be predicted $(s,?, o)$ . As such, the target relation types may be reduced to e.g., $R_T = R_{\tau(s),\tau(o)} \subset R$ and then the candidate triples may be reduced to a semantically plausible list as $\{(s,p,o)\forall p \in R_{\tau(s),\tau(o)}\}$, where the number of plausible relationship types is below the number of all relation types IRI. For example, in a relation prediction query *(MYH7, ?, beta (β)-myosin heavy chain),* the query triple may fit into <gene, encodes, protein> type of triple due to the entity type of given subject and object nodes of the query triple. Thus, the candidate generation circuitry 406 may directly delivers *(MYH7, encodes, beta (β)-myosin heavy chain)* as a semantically plausible candidate to the link predictor 406 for likelihood estimation.

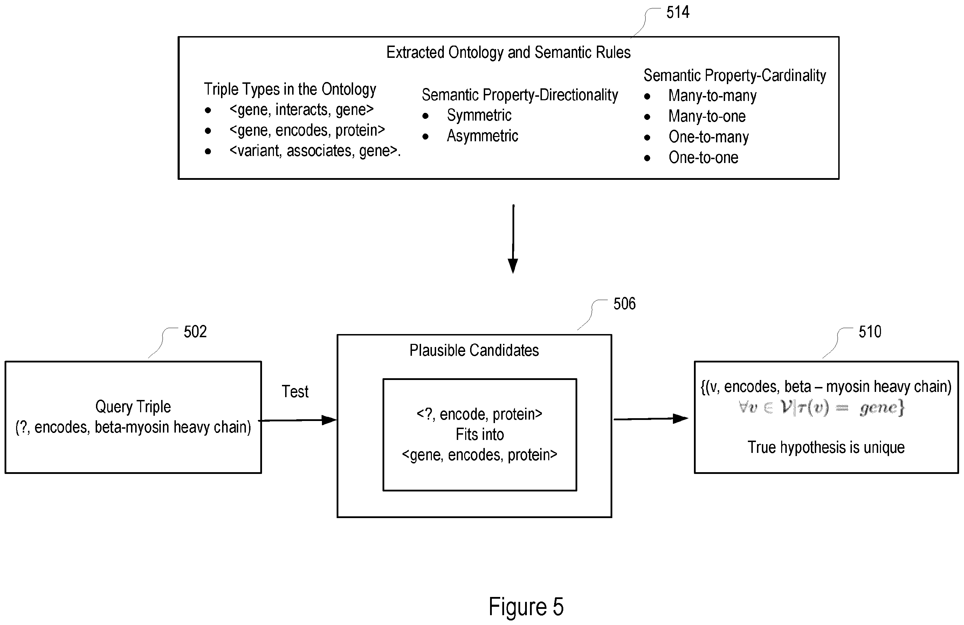

Further examples for the reduction of number of candidate triples by triple type are given below. In the link prediction query triple *(?, encodes, beta (β)-myosin heavy chain),* referring to Figure 5, the candidate generation circuitry may first identify which semantically plausible triple types that the link prediction query triple 502 fits into according to the extracted ontology and semantic rules 514. For this purpose, it checks the entity type of the given counterpart of the query triple and determines that the object *beta (β)-myosin heavy chain* belongs to the type of a protein, yielding a query triple type as <?, encodes, protein>, as shown by 506. By compare this query triple type against all triple types derived by the rule extraction circuitry 414, the candidate generation circuitry 406 determines that the query triple fits into <gene, encodes, protein> type of triple among other possible triple types, as shown by 506. As a result, the candidate generation circuitry 406 reduce the list target candidate subject nodes to nodes in the KG that are of the type gene. Accordingly, the set of candidates for further link prediction is reduced to $\{(v, encodes, beta(\beta) - myosin\ heavy\ chain) \forall v \in V | \tau(v) = gene\}$, which has a size (number of candidate target nodes) that is equal to the number of nodes of the gene type in the KG. For instance, while *(MYH7, encodes, beta (β)-myosin heavy chain)* is a candidate according to the candidate generation circuitry 406 because *MYH7* is a gene, the candidate generation circuitry 406 would eliminates the semantically invalid candidates including target node of type drug and others, which alleviates naive hallucinations at the output of the link predictor 408.

As described above, once the triple types that query triple (404) (e.g., a link prediction query triple (s, p, ?) or (?, p, o) or a relation prediction query triple (s, ?, o)) fits into are identified among all triple types, the number of triple candidates for processing by the link predictor 406 is already reduced by only including the plausible set of target nodes or target relation types. As a next example step, the candidate generation circuitry 406 may be configured to further reduce the candidate triple set based on the semantic properties of the fitting plausible triple types, including but not limited to directionality and cardinality properties. Example implementations are further provided in detail below.

In some example implementations, the directionality property generated from the rule extraction circuitry 414 may be employed by the candidate generation circuitry 406 to designate some candidates directly as true hypotheses, thereby bypassing the link prediction process all together. For example, in cases where the link prediction task involves query triple types (as generated from the input query triple 404) that are determined by the rule extraction circuitry 414 as symmetric triple types, it may be possible to guarantee some candidate triples to be true which may be provided directly as predicted links. Such triples may be considered as true hypothesis.

For example, a query triple *(HADH, interacts, ?)* may be determined by the candidate generation circuitry 406 as fitting the triple type of <gene, interact, gene>, which may have been determined as constituting a symmetric relation by checking the semantic rules attached to that triple type as extracted and provided by the rule extraction circuitry 414. As such, this query triple may be considered by the rule extraction circuitry 414 as being equivalent to its symmetric counterpart query triple *(?, interacts, HADH)* by symmetrically swapping the subject node and the object position in the original query triple. If the instance *(STAT1, interacts, HADH)* is already in the input KG, then the candidate generation circuitry delivers *(HADH, interacts, STAT1)* directly as a guaranteed predicted candidate triples under the query of *(HADH, interacts, ?)*. The likelihood calculation by the link predictor 406 may be omitted altogether, thereby greatly reducing the inference time for obtaining this hypothesis. The candidate generation circuitry 406 may identify other candidate triples which may still need to go through the processing by the link predictor 406 for likelihood estimation and ranking. For example, STAT1 may be identified as a gene, but neither of (*HADH, interacts, STAT1*) or (*STAT1, interacts, HADH*) triple instance is in the KG. Then the candidate (*HADH, interacts, STAT1*) would be processed by the link predictor circuitry 408 for likelihood estimation, and ranking.

In some example implementations, the cardinality property generated from the rule extraction circuitry 414 may be additionally or alternatively employed by the candidate generation circuitry 406 to further reduce the number of semantically plausible candidates for processing by the link predictor circuitry 408.

For example, in a link prediction task, it may be important to know the cardinality of the triple types fitting the input query triple to determine whether (1) the answer to the query is null; (2) the answer is unique; (3) the answer is subject to multiple target triples. If the answer is null, then there is no need to perform any link prediction because it is likely that there could not be any valid candidates/hypothesis. If the answer is unique, then there may be valid candidate triples but only one may be true. If the answer indicates that there are multiple target candidates, then multiple hypotheses may be true.

For another example, for triple types with singular cardinality, if a link prediction query such as (s, p,?) has a fitting triple type of singular object cardinality such as one-to-one and many-to-one, or if a link query such as (?,p, o) has a fitting triple type of singular subject cardinality such as one-to-one and one-to-many, then there cannot be multiple true hypotheses-the correct answer in hypotheses may be either null or unique.

In a particular example, an input query triple *(?, encodes, beta (β)-myosin heavy chain),* referring to 502 of Figure 5, may be determined by the candidate generation circuitry 406 as fitting the triple type *<gene, encodes, protein>,* which may be determined by the candidate generation circuitry 406 according to the rules 514 extracted by the rule extraction circuitry 414 to be asymmetric and one-to-many type of triple. This means that there could be only one node of gene type connecting to the given object node through relation type 'encodes'. If in the input KG there is no instance of such triple type where the object is *beta (β)-myosin heavy chain,* then the candidate generation circuitry 406 would deliver a candidate list that is composed of gene type of subject nodes to the link predictor circuitry 408 after reduction to semantically plausible target node, with the information that the true hypothesis is unique with the goal to predict the single Gene that encodes the protein *beta (β)-myosin heavy chain.* Such information may be passed along to the human end-user and can be valuable for the human end user, who may evaluate the accuracy of hypotheses set manually by considering such information. In cases where an instance of such triple type where the object is *beta (β)-myosin heavy chain* is found in the input KG e.g., *(MYH7, encodes, beta (β)-myosin heavy chain),* that instance may be directly delivered as a factual response to the user, bypassing any likelihood estimation by the link predictor circuity 408 and reducing inference time.

In some other example cases, the triple types fitting the input query triple may be associated with plural cardinality, e.g., there can be multiple true hypothesis for a link prediction query (s, p, ?) fitting with triple types having plural object cardinality or in a query (?, p, o) fitting with triple types having plural subject cardinality. Although this would not reduce the candidate list or give some guaranteed candidates/hypothesis to be true, this information can still be delivered to the end user in order to indicate that multiple hypothesis during testing through the link predictor circuitry 408 may be true.

As described in more detail above, in some example implementations, a computer-implemented method for predicting unknown triples in an input knowledge graph is implemented. The method may include receiving the input knowledge graph; automatically obtaining an ontology of the input knowledge graph; generating a list of reference triple types for the input knowledge graph based on the ontology; automatically extracting a set of semantic rules associated with the input knowledge graph; receiving a query triple instance; extracting a query triple type corresponding to the query triple instance, and a set of semantic rules based on the input knowledge graph and the ontology; generating a set of candidate unknown triples by expanding the query triple instance based on and as restricted by the query triple type, the input knowledge graph, and the set of semantic rules; and automatically generating a ranked list of predicted unknown triples from the set of candidate unknown triples using a pretrained link prediction circuitry.

In the example implementation above, the ranked list predicted triples comprises at least one triple including a node indicating a specific gene, and the method further comprises subject the gene to a wet lab testing for experimental evaluation according to the at least one triple.

In any one of the example implementations above, the ranked list of predicted triples comprises at least one triple including a node indicating a specific gene, and the method further comprises subject a physical entity of the specific gene to a wet lab testing for experimental evaluation according to the at least one triple.

In any one of the example implementations above, the ontology is automatically extracted from the input knowledge graph or based on a schema from which the input knowledge graph is instantiated.

In any one of the example implementations above, the query triple instance comprises a placeholder for one of a subject node, object node, and predicate.

In any one of the example implementations above, the query triple instance comprises the subject node, the predicate, and the placeholder for the object node; or the object node, the predicate, and the placeholder for the subject node; or the subject node, the object node, and the placeholder for the predicate.

In any one of the example implementations above, the query triple type is extracted by converting the subject node, object node, and the predicate into corresponding entity types or predicates except the placeholder.

In any one of the example implementations above, generating the set of candidate triples may include identifying a set of plausible entity or predicate types for the placeholder of the query triple instance based on the query triple type and the list of reference triple types of the input; and generating the set of candidate triples by expanding the placeholder of the query triple instance with a set of nodes or predicates of the input knowledge graph that conform with the set of plausible entity or predicate types for the placeholder.

In any one of the example implementations above, the set of semantic rules may include at least one of a directionality and a cardinality of each triple type of the list of reference triple types for the input knowledge graph.

In any one of the example implementations above, the directionality for each triple type of the list of reference triple types indicates whether each triple type is reciprocal with respect to a subject and an object within each triple type.

In any one of the example implementations above, the directionality is derived from a combination of the ontology and the input knowledge graph.

In any one of the example implementations above, the cardinality for each triple type of the list of reference triple types indicates a multiplicity of a subject instance and/or an object instance within each triple type.

In any one of the example implementations above, the cardinality is derived by a counting procedure of a first number of triple instances a second number of unique subject instances and a third number of unique object instances associated with each triple type.

In any one of the example implementations above, the directionality is used at least to bypass the pretrained link prediction circuitry to generate at least one direct unknown triple that is considered as a true triple of the input knowledge graph.

In any one of the example implementations above, the cardinality is used at least to bypass the pretrained link prediction circuitry to generate at least one direct unknown triple that is considered as a true triple of the input knowledge graph.

In any one of the example implementations above, the list of reference triple types for the input knowledge graph is further reduced using a counting procedure performed on the input knowledge graph and removing triple types with few triple instances that can be counted as outlier or error.

In some other example implementations, a system for predicting unknown triples in a n input knowledge graph is disclosed. The system may include a memory for storing instructions, and a processor in communication with the memory, wherein the processor, when executing the instructions, is configured to implement any one of the methods above.

In yet some other example implementations, a non-transitory computer-readable medium is disclosed, including instructions configured to be executed by a processor, wherein the instructions are adapted to cause the processor to predict unknown triples in an input knowledge graph using any one of the methods above.

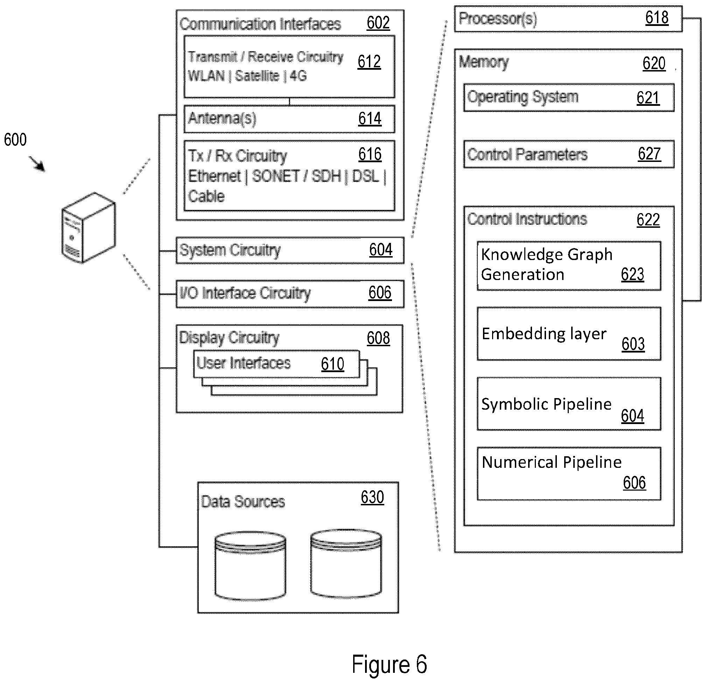

Finally, Figure 6 illustrates a computer architecture of a computer device 600 for generating the candidate triples, for deriving the ontology and semantic rules, and for the link prediction. The computer device 600 includes communication interfaces 602, system circuitry 604, input/output (I/O) interface circuitry 606, and display circuitry 608. The graphical user interfaces (GUIs) 610 displayed by the display circuitry 608 may be representative of GUIs generated by the knowledge graph prediction system to present or request information. The GUIs 610 may be displayed locally using the display circuitry 608, or for remote visualization, e.g., as HTML, JavaScript, audio, and video output for a web browser running on a local or remote machine.

The GUIs 610 and the I/O interface circuitry 606 may include touch sensitive displays, voice or facial recognition inputs, buttons, switches, speakers and other user interface elements. Additional examples of the I/O interface circuitry 606 includes microphones, video and still image cameras, headset and microphone input / output jacks, Universal Serial Bus (USB) connectors, memory card slots, and other types of inputs. The I/O interface circuitry 606 may further include magnetic or optical media interfaces (e.g., a CDROM or DVD drive), serial and parallel bus interfaces, and keyboard and mouse interfaces.

The communication interfaces 602 may include wireless transmitters and receivers (herein, "transceivers") 612 and any antennas 614 used by the transmit-and-receive circuitry of the transceivers 612. The transceivers 612 and antennas 614 may support WiFi network communications, for instance, under any version of IEEE 802.11, e.g., 802.11n or 802.11ac, or other wireless protocols such as Bluetooth, Wi-Fi, WLAN, cellular (4G, LTE/A). The communication interfaces 602 may also include serial interfaces, such as universal serial bus (USB), serial ATA, IEEE 1394, lighting port, $I^2C$, slimBus, or other serial interfaces. The communication interfaces 602 may also include wireline transceivers 616 to support wired communication protocols. The wireline transceivers 616 may provide physical layer interfaces for any of a wide range of communication protocols, such as any type of Ethernet, optical networking protocols, data over cable service interface specification (DOCSIS), digital subscriber line (DSL), Synchronous Optical Network (SONET), or other protocol.

The system circuitry 604 may include any combination of hardware, software, firmware, APIs, and/or other circuitry. The system circuitry 604 may be implemented, for example, with one or more systems on a chip (SoC), application specific integrated circuits (ASIC), microprocessors, discrete analog and digital circuits, and other circuitry. The system circuitry 604 may implement any desired functionality for generating the candidate triples, for deriving the ontology and semantic rules, and for the link prediction. As just one example, the system circuitry 604 may include one or more instruction processor 618 and memory 620.

The memory 620 stores, for example, control instructions 622 for executing the features of the knowledge graph prediction system, as well as an operating system 621. In one implementation, the processor 618 executes the control instructions 622 and the operating system 621 to carry out any desired functionality for generating the candidate triples, for deriving the ontology and semantic rules, and for the link prediction. The memory 620 additionally stores control instructions 622 for data processing and analytics 623 needed for generating the candidate triples, for deriving the ontology and semantic rules, and for the link prediction.

The computer device 600 may further include various data sources 630. Each of the databases that are included in the data sources 630 may be accessed by the candidate generation, ontology and semantic rule extraction, and link prediction system to obtain data for consideration during any one or more of the processes described herein.

The present disclosure may be embodied in various forms, including a system, a method, a computer readable medium, or a platform-as-a-service (PaaS) product for CLAIMS

While the present disclosure has been particularly shown and described with reference to the example embodiments thereof, it will be understood by those skilled in the art that various changes in form and details may be made therein without departing from the spirit and scope of the present disclosure. Although some of the drawings illustrate a number of operations in a particular order, operations that are not order-dependent may be reordered and other operations may be combined or broken out. While some reordering or other groupings are specifically mentioned, others will be apparent to those of ordinary skill in the art and so do not present an exhaustive list of alternatives.

**NUMBERED STATEMENTS OF INVENTION**

1. A computer-implemented method for predicting unknown triples in an input knowledge graph, comprising:

receiving the input knowledge graph;

automatically obtaining an ontology of the input knowledge graph; generating a list of reference triple types for the input knowledge graph based on the ontology;
automatically extracting a set of semantic rules associated with the input knowledge graph;
receiving a query triple instance;

extracting a query triple type corresponding to the query triple instance, and a set of semantic rules based on the input knowledge graph and the ontology;

generating a set of candidate triples by expanding the query triple instance based on and as restricted by the query triple type, the input knowledge graph, and the set of semantic rules; and
automatically generating a ranked list of predicted unknown triples from the set of candidate triples using a pretrained link prediction circuitry.

2. The computer-implemented method of statement 1, where the ranked list of predicted unknown triples comprises at least one triple including a node indicating a specific gene, and the method further comprises subject a physical entity of the specific gene to a wet lab testing for experimental evaluation according to the at least one triple.

3. The computer-implemented method of statement 1, wherein the ontology is automatically extracted from the input knowledge graph.

4. The computer-implemented method of statement 1, wherein the ontology is extracted based on a schema from which the input knowledge graph is instantiated.

5. The computer-implemented method of statement 1, wherein the query triple instance comprises a placeholder for one of a subject node, object node, and a predicate.

6. The computer-implemented method of statement 5, wherein the query triple instance comprises the subject node, the predicate, and the placeholder for the object node.

7. The computer-implemented method of statement 5, wherein the query triple instance comprises the object node, the predicate, and the placeholder for the subject node.

**8.** The computer-implemented method of statement 5, wherein the query triple instance comprises the subject node, the object node, and the placeholder for the predicate.

**9.** The computer-implemented method of statement 5, wherein the query triple type is extracted by converting the subject node, object node, and the predicate into corresponding entity types or predicate types except the placeholder.

**10.** The computer-implemented method of statement 5, wherein generating the set of candidate triples comprises:

identifying a set of plausible entity or predicate types for the placeholder of the query triple instance based on the query triple type and the list of reference triple types of the input knowledge graph; and
generating the set of candidate triples by expanding the placeholder of the query triple instance with a set of nodes or predicates of the input knowledge graph that conform with the set of plausible entity or predicate types for the placeholder.

**11.** The computer-implemented method of statement 10, wherein the set of semantic rules comprise at least one of a directionality and a cardinality of each triple type of the list of reference triple types for the input knowledge graph.

**12.** The computer-implemented method of statement 11, wherein the directionality for each triple type of the list of reference triple types indicates whether each triple type is
reciprocal with respect to a subject and an object within each triple type.

**13.** The computer-implemented method of statement 12, where the directionality is derived from a combination of the ontology and the input knowledge graph.

**14.** The computer-implemented method of statement 12, wherein the cardinality for each triple type of the list of reference triple types indicates a multiplicity of a subject instance and/or an object instance within each triple type.

**15.** The computer-implemented method of statement 14, where the cardinality is derived by a counting procedure of a first number of triple instances a second number of unique subject instances and a third number of unique object instances associated with each triple type.

**16.** The computer-implemented method of statement 14, wherein the directionality is used
at least to bypass the pretrained link prediction circuitry to generate at least one direct unknown triple that is considered as a true triple of the input knowledge graph.

**17.** The computer-implemented method of statement 14, wherein the cardinality is used at least to bypass the pretrained link prediction circuitry to generate at least one direct unknown triple that is considered as a true triple of the input knowledge graph.

**18.** The computer-implemented method of statement 14, wherein the list of reference triple types for the input knowledge graph is further reduced using a counting procedure performed on the input knowledge graph and removing triple types with few triple instances that can be counted as outlier or error.

**19.** A system for predicting unknown triples in a knowledge graph, the system comprising a memory for storing instructions, and a processor in communication with the memory, wherein the processor, when executing the instructions, is configured to:

receive an input knowledge graph;
automatically obtain an ontology of the knowledge graph;
generate a list of reference triple types for the knowledge graph based on the ontology;
automatically extract a set of semantic rules associated with the knowledge graph;
receive a query triple instance;

extract a query triple type corresponding to the query triple instance, and a set of semantic rules based on the input knowledge graph and the ontology;
generate a set of candidate triples by expanding the query triple instance based on and as restricted by the query triple type, the input knowledge graph, and the set of semantic rules; and

automatically generate a ranked list of predicted unknown triples from the set of candidate triples using a pretrained link prediction circuitry.

**20.** A non-transitory computer-readable medium including instructions configured to be executed by a processor, wherein the instructions are adapted to cause the processor to predict unknown triples in an input knowledge graph by:
receiving the input knowledge graph;

automatically obtaining an ontology of the input knowledge graph;
generating a list of reference triple types for the input knowledge graph based on the ontology;
automatically extracting a set of semantic rules associated with the input knowledge graph;
receiving a query triple instance;
extracting a query triple type corresponding to the query triple instance, and a set of semantic rules based on the input knowledge graph and the ontology;

generating a set of candidate triples by expanding the query triple instance based on and as restricted by the query triple type, the input knowledge graph, and the set of semantic rules; and
automatically generating a ranked list of predicted unknown triples from the set of candidate triples using a pretrained link prediction circuitry.

**Claims**

1. A computer-implemented method for predicting unknown triples in an input knowledge graph, comprising:

   receiving the input knowledge graph;
   automatically obtaining an ontology of the input knowledge graph;
   generating a list of reference triple types for the input knowledge graph based on the ontology;

   automatically extracting a set of semantic rules associated with the input knowledge graph;
   receiving a query triple instance;

   extracting a query triple type corresponding to the query triple instance, and a set of semantic rules based on the input knowledge graph and the ontology;

   generating a set of candidate triples by expanding the query triple instance based on and as restricted by the query triple type, the input knowledge graph, and the set of semantic rules; and
   automatically generating a ranked list of predicted unknown triples from the set of candidate triples using a pretrained link prediction circuitry.

2. The computer-implemented method of claim 1, where the ranked list of predicted unknown triples comprises at least one triple including a node indicating a specific gene, and the method further comprises subject a physical entity of the specific gene to a wet lab testing for experimental evaluation according to the at least one triple.

3. The computer-implemented method of claim 1 or 2, wherein

   the ontology is automatically extracted from the input knowledge graph and/or
   wherein the ontology is extracted based on a schema from which the input knowledge graph is instantiated.

4. The computer-implemented method of any one of the previous claims, wherein the query triple instance comprises a placeholder for one of a subject node, object node, and a predicate.

5. The computer-implemented method of any one of the previous claims, wherein

   the query triple instance comprises the subject node, the predicate, and the placeholder for the object node and/or
   wherein the query triple instance comprises the object node, the predicate, and the placeholder for the subject node and/or
   wherein the query triple instance comprises the subject node, the object node, and the placeholder for the predicate and/or

wherein the query triple type is extracted by converting the subject node, object node, and the predicate into corresponding entity types or predicate types except the placeholder.

6. The computer-implemented method of any one of the previous claims, wherein generating the set of candidate triples comprises:

identifying a set of plausible entity or predicate types for the placeholder of the query triple instance based on the query triple type and the list of reference triple types of the input knowledge graph; and
generating the set of candidate triples by expanding the placeholder of the query triple instance with a set of nodes or predicates of the input knowledge graph that conform with the set of plausible entity or predicate types for the placeholder.

7. The computer-implemented method of any one of the previous claims, wherein the set of semantic rules comprise at least one of a directionality and a cardinality of each triple type of the list of reference triple types for the input knowledge graph.

8. The computer-implemented method of claim 7, wherein the directionality for each triple type of the list of reference triple types indicates whether each triple type is reciprocal with respect to a subject and an object within each triple type.

9. The computer-implemented method of claim 7 or 8, where the directionality is derived

from a combination of the ontology and the input knowledge graph and/or wherein the cardinality for each triple type of the list of reference triple types indicates a multiplicity of a subject instance and/or an object instance within each triple type.

10. The computer-implemented method of any one of claims 7 to 9, where the cardinality is derived by a counting procedure of a first number of triple instances a second number of unique subject instances and a third number of unique object instances associated with each triple type.

11. The computer-implemented method of any one of claims 7 to 10, wherein the directionality is used at least to bypass the pretrained link prediction circuitry to generate at least one direct unknown triple that is considered as a true triple of the input knowledge graph.

12. The computer-implemented method of any one of claims 7 to 11, wherein the cardinality is used at least to bypass the pretrained link prediction circuitry to generate at least one direct unknown triple that is considered as a true triple of the input knowledge graph.

13. The computer-implemented method of any one of the previous claims, wherein the list of reference triple types for the input knowledge graph is further reduced using a counting procedure performed on the input knowledge graph and removing triple types with few triple instances that can be counted as outlier or error.

14. A system for predicting unknown triples in a knowledge graph, the system comprising a memory for storing instructions, and a processor in communication with the memory, wherein the processor, when executing the instructions, is configured to:

receive an input knowledge graph;
automatically obtain an ontology of the knowledge graph;
generate a list of reference triple types for the knowledge graph based on the ontology;
automatically extract a set of semantic rules associated with the knowledge graph;
receive a query triple instance;

extract a query triple type corresponding to the query triple instance, and a set of semantic rules based on the input knowledge graph and the ontology;
generate a set of candidate triples by expanding the query triple instance based on and as restricted by the query triple type, the input knowledge graph, and the set of semantic rules; and

automatically generate a ranked list of predicted unknown triples from the set of candidate triples using a pretrained link prediction circuitry.

15. A computer-readable medium including instructions configured to be executed by a processor, wherein the instructions are adapted to cause the processor to predict unknown triples in an input knowledge graph by:
receiving the input knowledge graph;

automatically obtaining an ontology of the input knowledge graph;
generating a list of reference triple types for the input knowledge graph based on the ontology;
automatically extracting a set of semantic rules associated with the input knowledge graph;
receiving a query triple instance;
extracting a query triple type corresponding to the query triple instance, and a set of semantic rules based on the input knowledge graph and the ontology;

generating a set of candidate triples by expanding the query triple instance based on and as restricted by the query triple type, the input knowledge graph, and the set of semantic rules; and
automatically generating a ranked list of predicted unknown triples from the set of candidate triples using a pretrained link prediction circuitry.

Figure 1

EP 4 495 799 A1

Figure 2

300

302 Input KG

Train

304 A Query Triple (s, p, ?)

Test

306 Candidate Generation Circuitry

308 Link Prediction Circuitry

Likelihood Computation and Ranking

310 List of Hypothesis

Figure 3

Figure 4

514

Extracted Ontology and Semantic Rules

Triple Types in the Ontology
- <gene, interacts, gene>
- <gene, encodes, protein>
- <variant, associates, gene>.

Semantic Property-Directionality
- Symmetric
- Asymmetric

Semantic Property-Cardinality
- Many-to-many
- Many-to-one
- One-to-many
- One-to-one

506

502

Query Triple
(?, encodes, beta-myosin heavy chain)

Test

Plausible Candidates

<?, encode, protein>
Fits into
<gene, encodes, protein>

510

$\{(v, \text{encodes}, \text{beta} - \text{myosin heavy chain})$
$\forall v \in \mathcal{V} | \tau(v) = gene\}$

True hypothesis is unique

Figure 5

Figure 6

EP 4 495 799 A1

Europäisches
Patentamt

European
Patent Office

Office européen
des brevets

# EUROPEAN SEARCH REPORT

**Application Number**

EP 24 18 5540

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 404 568 B1 (FUJITSU LTD [JP]) 12 July 2023 (2023-07-12) * the whole document * ----- | 1-15 | INV. G06F16/36 G06F16/901 G16H50/70 G16H50/80 G06N5/025 G06N20/00 |

**TECHNICAL FIELDS SEARCHED (IPC)**

G06F
G16H
G06N

The present search report has been drawn up for all claims

| Place of search | Date of completion of the search | Examiner |
|---|---|---|
| The Hague | 6 December 2024 | Ntarlagiannis, V |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...........................................................................

& : member of the same patent family, corresponding
document

EPO FORM 1503 03.82 (P04C01)

**ANNEX TO THE EUROPEAN SEARCH REPORT
ON EUROPEAN PATENT APPLICATION NO.**                    EP 24 18 5540

This annex lists the patent family members relating to the patent documents cited in  the above-mentioned European search report.
The members are as contained in the European Patent Office EDP file on
The European Patent Office is in no way liable for these particulars which are merely given for the purpose of information.

06-12-2024

| Patent document cited in search report | | Publication date | Patent family member(s) | | Publication date |
|---|---|---|---|---|---|
| EP 3404568 | B1 | 12-07-2023 | EP | 3404567 A1 | 21-11-2018 |
| | | | EP | 3404568 A1 | 21-11-2018 |
| | | | JP | 7155604 B2 | 19-10-2022 |
| | | | JP | 2018206374 A | 27-12-2018 |

EPO FORM P0459

For more details about this annex : see Official Journal of the European Patent Office, No. 12/82